# EUROPÄISCHE PATENTSCHRIFT

(11) **EP 2 283 802 B1**
(45) Veröffentlichungstag und Bekanntmachung des Hinweises auf die Patenterteilung: **20.06.2012**
(21) Anmeldenummer: 10007478.0
(22) Anmeldetag: 20.07.2010
(51) Int. Cl.: A61K 6/00, A61K 8/22, A61K 8/92, A61Q 11/00, A61K 9/00

(54) **Medizinisches Präparat zur Behandlung von entzündlichen Prozessen**
Medicinal preparation for treating inflammatory processes
Préparation médicale pour le traitement de processus inflammatoires

(30) Priorität: 21.07.2009 DE 102009034308
(43) Veröffentlichungstag der Anmeldung: 16.02.2011
(73) Patentinhaber: Winkelmann, Wolfgang, 72076 Tübingen (DE)
(72) Erfinder: Winkelmann, Wolfgang, 72076 Tübingen (DE)
(74) Vertreter: Wüstefeld, Regine Marie

(56) Entgegenhaltungen:
- EP-A1- 1 935 408
- WO-A1-97/21417
- DE-A1- 19 932 570
- US-A1- 2005 026 107

## Beschreibung

Die Erfindung betrifft ein medizinisches Präparat zur Behandlung von entzündlichen Prozessen, insbesondere von entzündlichen Prozessen in der Mundhöhle, ein Verfahren zur Herstellung eines Trägers für das medizinische Präparat, den so hergestellten Träger selbst und eine besondere Ausführungsform des medizinischen Präparats, das diesen Träger aufweist.

Als eine Entzündung in der Mundhöhle ist die Parodontitis weit verbreitet. Diese wird durch Bakterien verursacht, welche dann, wenn keine Behandlung erfolgt, nach und nach zu einer Zerstörung des Zahnhalteapparates führen, die nicht mehr rückgängig zu machen ist. Zahnverluste sind die Folge.
Eine Möglichkeit, solche bakteriellen Infektionen zu bekämpfen, besteht in der lokalen Anwendung breitbandiger oder spezifisch wirksamer Antibiotka. Bekannt in diesem Zusammenhang ist die Anwendung des Antibiotikums Doxycyclin aus der Klasse der Tetracycline, das ein breitbandiges Wirkungsspektrum aufweist.

Nachteilig ist jedoch bei der Verwendung dieses oder eines anderen Antibiotikurns, daß es wiederholt angewendet werden muß. In der Zahlheilkunde hat sich in bezug auf die Parodontitis die Erkenntnis durchgesetzt, daß eine einmalige Behandlung nicht ausreichend ist. Insbesondere bei einer genetischen Disposition bedarf es oft lebenslang durchgeführter Wiederholungsbehandlungen, um die durch eine Parodontitis in Gang gesetzte Zerstörung des Zahnhalteapparates wirksam aufzuhalten. Diese wiederholten Behandlungen können zu einer Antibiotikum-Resistenz und damit auf Dauer zu einer Unwirksamkeit der Behandlung führen.

Als eine Möglichkeit, Wiederholungsbehandlungen mit einem Antibiotikum zu vermeiden, ist im Stand der Technik vorgeschlagen worden, eine kleine Membran in Form einer Gelatinefolie in die betroffenen Zahafleischtaschen einzusetzen, die mit Chlorhexidin versetzt ist. Die Membran löst sich zeitverzögert selbstständig auf.
Der verwendete Wirkstoff Chlorhexidin wird seit langem als Antiseptikum in der Mundhöhle über einen in der Regel begrenzten Zeitraum eingesetzt. Auch wenn dieser sich über einige Wochen hinziehen kann, ist dies im Vergleich zu einer jahre- oder lebenslangen Verwendung ein überschaubarer kurzer Zeitraum. Gegen eine Verwendung des Wirkstoffs Chlorhexidin in der Langzeittherapie sprechen die Nebenwirkungen, die von diesem Wirkstoff aufgrund der chlorhaltigen Formulierung bekannt sind. Dazu gehört neben einer Störung des Geschmacksempfindens auch die Tatsache, daß sogar eine verzögerte Wundheilung beobachtet werden kann. Zu den eher ästhetischen Nachteilen bei der Verwendung des Wirkstoffs Chlorhexidin gehört die bei dessen Anwendung zu beobachtende Bildung von bräunlichen Ablagerungen an den umgebenden Zähnen und teilweise auch auf der Zunge.

Auch die als Grundlage der Membran verwendete Gelatine ist heute durch die verschiedenen Erkrankungen von Rind und Schwein und den verstärkten Trend zu einer vegetarischen Lebensweise nicht mehr unumstritten und wird vielfach abgelehnt.

Die deutsche Offenbarungsschrift DE 199 32 570 beschreibt ein Präparat zur antimikrobiellen Behandlung im Zahnbereich (Paradontosebehandlung), welches ein mit Ozon angereichertes Oliven- und/oder Rizinusöl enthält, das mit Hilfe von Polyethylenglykol in der Konsistenz eingestellt wird.

Die europäische Schrift EP 1 935 408 offenbart ebenfalls ein Präparat zur Behandlung von Zähnen und Entzündungen im Mund, welche mit Ozon angereicherte Pflanzenöle (Sonnenblumenkernöl, Olivenöl) enthalten.

Die US 2005/026107 offenbart ein therapeutisches Objekt zur Behandlung von Krankheiten im Mundraum bestehend aus einem Träger aus PVP oder Cellulose jedoch nicht Polysacchariden. Als Wirkstoff kommen Peroxide und Wasserstoffperoxid in Frage, die nicht in Pflanzenöl aufgenommen sind.

Ausgehend von diesem Stand der Technik lag der vorliegenden Erfindung daher die Aufgabe zugrunde, ein nichtantibiotisches medizinisches Präparat bereitzustellen, das als Langzeittherapeutikum bei der Behandlung von entzündlichen Prozes-sen, und insbesondere im Bereich der Zahlkeilkunde bei der Behandlung von Parodontitis, auch bei Patienten mit vegetarischer oder veganer Lebensführung eingesetzt werden kann.

Gelöst wird diese Aufgabe durch ein medizinisches Präparat, bei dem ein antiphlogistisches Mittel in Form von ozonisiertem Pflanzenöl und/oder dessen Bestandteilen auf einem Trägermaterial auf pflanzlicher und/oder Kunststoffbasis aufgebracht ist, und das mit einem polymeren Überzug oder einem Überzug aus einem natürlichen oder synthetischen Wachs versehen ist, wobei der Träger und der Überzug im wesentlichen biologisch abbaubar sind.

Der biologisch abbaubare Träger ist vorzugsweise auf der Basis von Polysacchariden aufgebaut. Zu den Polysacchariden werden die Kohlehydrate gezählt. Insbesondere Mehl verschiedener Getreidearten und Ausmahlungsgrade kann dabei als Grundlage für den Träger dienen. Mehle dieser Art haben grundsätzlich einen Kohlehydratanteil von ca. 60 - 75%. Zu den Kohlehydraten zählt ebenso die Stärke. Auch Cellulose ist als ein Polysaccharid zu nennen. Entsprechend kann das Trägermaterial auf der Basis von Stoffen aufgebaut sein, die ausgewählt sind aus Stärke unterschiedlicher Herkunft, Cellulose, Cellulosedenvaten, Polyglycoliden (PGA) und deren Copolymeren, Polylactid-co-glycoliden (PLGA), Polyanhydriden, Polyorthoestern, Vinylpolymeren, Polyvinylpyrrolidon und Mischungen davon.
Als ein besonders einfacher Grundstoff auf der Basis von Polysacchariden kann ein Träger in Form von herkömmlichem Eßpapier, wie es z.B. für Obladen benötigt wird, verwendet werden.

Cellulose als Polysaccharid ist zwar grundsätzlich ein vom menschlichen Körper nicht verdaubarer Stoff, doch der erfindungsgemäß Cellulose aufweisende Träger ist z.B. in der Umgebung der Mundschleimhaut mit den dort wirkenden Enzymen nicht formbeständig, löst seine Form auf und die resultierenden Bestandteile werden dem Stoffwechsel als Ballaststoffe zugeführt.

Unter Cellulosederivaten wird in der Regel eine künstlich, d.h. durch chemische Umsetzungen veränderte Cellulose verstanden, die so in der Natur nicht vorkommt. Hier sind als verwendbare Beispiele Celluloseether, Methylcellulose, Hydroxyproylcellulose und Hydroxyproylmethylcellulose zu nennen.

Polyglycolide oder Polyglykolsäure sind bioabbaubare thermoplastische Polymere, die aufgrund ihrer hydrolytischen Abbaubarkeit für die Zwecke der vorliegenden Erfindung gut eignet sind. In diesem Zusammenhang sind auch noch die entsprechenden, auf der Basis von Glykolsäure gebildeten Copolymere zu nennen. Als Copolymere kommen insbesondere Polylactid-co-glycolid in Verbindung mit Lactonsäure, Polylactid-co-caprolactam in Verbindung mit ε-Caprolactam oder Polylactid-co-trimetylencarbonat in Verbindung mit Trimethylencarbonat in Frage.

Von Polyvinylpyrrolidon ist die Verwendung in der pharmazeutischen Industrie hinlänglich bekannt. Polyvinylpyrrolidon wird z. B. in Tabletten als Bindemittel eingesetzt. Erfindungsgemäß kann Polyvinylpyrrolidon auch als ein Trägermaterial eingesetzt werden, in Verbindung mit einem geeigneten polymeren Überzug. In ersten Versuchen hat sich gezeigt, daß das antiphlogistische Mittel dann sogar eine Vernetzung mit dem Trägerstoff zeigt.

Gemäß einer Ausführungsform des erfindungsgemäßen medizinischen Präparats ist das das ozonisierte Pflanzenöl ausgewählt aus Rapsöl, (auch Rüböl oder Rüpsenöl genannt), Sonnenblumenkernöl, Sojaöl, Rizinusöl, Olivenöl, Leinöl, Kokosöl, Palmöl sowie zumindest ein Bestandteil, Derivat und/oder Mischungen davon zu nennen.
Es soll aber an dieser Stelle darauf hingewiesen werden, daß es sich dabei um eine exemplarische Aufzählung der gängigen Pflanzenöle handelt, und daß grundsätzlich jegliches Pflanzenöl für die Zwecke der vorliegenden Erfindung eingesetzt werden kann.

Das Derivat ist ein Ester vorzugsweise ein Glycerinester einer mehrfach ungesättigten Fettsäure.

Es ist hinlänglich bekannt, daß die natürlichen pflanzlichen Öle Glycerinester der höheren geradzahligen Fettsäuren, d. h. der Glyceride darstellen und bis zu ca. 97% aus Triglyceriden, bis zu ca. 3% aus Diglyceriden und bis ca. 1% aus Monoglyceriden aufgebaut sind. Tri-, Di- und Monoglyceride bestehen jeweils aus einem Molekül Glycerin, verestert mit 3 Molekülen, 2 Molekülen bzw. 1 Molekül Fettsäure. Die chemischen, physikalischen und biologischen Eigenschaften der Pflanzenöle werden von der Art der Fettsäurekomponente und ihrer Verteilung über die Triglyceridmoleküle bestimmt. Es ist außerdem hinlänglich bekannt, daß der Schmelzpunkt im allgemeinen mit zunehmendem Anteil an langkettigen Fettsäuren bzw. mit abnehmendem Anteil an kurzkettigen oder ungesättigten Fettsäuren steigt. Die Eigenschaften eines Triglycerids werden auch durch die Stellung der verschiedenen Fettsäuregruppen innerhalb des Triglyceridmoleküls bestimmt.

Die Fettsäuren selbst sind überwiegend geradzahlige, geradkettige, aliphatische Monocarbonsäuren mit Kettenlängen von C₄ bis C₂₄. Der Schmelzpunkt einer Fettsäure fällt mit abnehmender Kettenlänge und zunehmender Anzahl von Doppelbindungen. Pflanzenöle sind bei Zimmertemperatur flüssig wegen ihres erheblichen Anteils an ungesättigten Fettsäuren.

Wenn das jeweilige Pflanzenöl als Ester der jeweiligen mehrfach ungesättigten Fettsäuren eingesetzt wird, sind grundsätzlich die Methylester von besonderem Interesse. Hier sind insbesondere Rapsfettsäuremethylester, Rizinolsäuremethylester, Ölsäuremethylester, Linolsäuremethylester, Laurinsäuremethylester zu nennen, wobei diese Aufzählung lediglich exemplarisch ist. Die Möglichkeiten sind mannigfaltig und die Auswahl sowie die gegebenenfalls weitere Umsetzung des pflanzlichen Öls oder seiner Bestandteile mit weiteren Fettsäuren ist für den Fachmann aufgrund seines allgemeinen Fachwissens ohne weiteres möglich.

Ozonisiertes Pflanzenöl in Form von ozonisiertem Olivenöl ist bereits bekannt. Es wird als solches, in Kapseln oder in Form von Salben angeboten. Als Anwendungsgebiete werden äußerliche Einreibungen mit dem ozonisierten Olivenöl bei Gelenkschmerzen und Rückenschmerzen, multipler Sklerose und Akne genannt. Bei Neurodermitis und Psoriasis wird eine kombinierte äußerliche Behandlung mit kolloidalem Silber vorgeschlagen, wobei beides nacheinander verwendet wird.

Das Ozon weist üblicherweise aufgrund der Art seiner Herstellung in einem Ozongenerator Sauerstoff als Trägergas auf. Entsprechend weist das in dem Pflanzenöl enthaltene Ozon seinerseits Sauerstoff als Trägergas auf. Aufgrund dieses Sauerstoffgehalts ist es nicht erforderlich, dem ozonisierten Pflanzenöl ein Konservierungsmittel hinzuzufügen. Außerdem wird die Wundheilung durch den anwesenden Sauerstoff gefördert.
Zu einem gewissen Anteil werden auch Peroxide ausgebildet. Mit dem Gehalt an aktivem Sauerstoff, der in dieser peroxidischen Form gebunden ist, läßt sich die Wirksamkeit des ozonisierten Pflanzenöls bzw. die Wirksamkeit von dessen Bestandteilen noch steigern.

Alternativ kann das antiphlogistische Mittel auch ausgewählt sein aus PVP-Jod. PVP-Jod wird handelüblich auch als Povidon-Jod bezeichnet, mit einem Gehalt von 10% an verfügbarem Jod in der Lösung.

Vorzugsweise sind die Abbaugeschwindigkeit des Trägers und des Überzugs jeweils so ausgewählt sind, daß sie unterschiedlich sind. Dabei ist es bevorzugt, daß der Überzug eine geringere Abbaugewhwindigkeit aufweist als der Träger.

Der polymere Überzug kann ausgewählt sein aus einem natürlichen Wachs, Cellulosederivaten, Polyethylenglykol, (Meth-)acrylsäurecopolymeren und/oder Mischungen davon sowie gegebenenfalls weiteren Hilfsstosen.

Als geeignete Vertreter der natürlichen Wachse sind z. B. Karnaubawachs, Candelilawachs, Japanwachs, Espartograswachs, Korkwachs und Guarumawachs zu nennen. Die Auswahl erfolgt vorzugsweise über ihre Verfügbarkeit und ihren Preis.
Karnaubawachs ist ein natürliches Wachs und als solches ein Lebensmittelzusatzstoff, der mit der Nummer E 903 gekennzeichnet ist. Er hat sich als Überzugsmittel für das erfindungsgemäße medizinische Präparat als sehr geeignet erwiesen. Besonders günstig ist sein hoher Schmelzpunkt, der zwischen 80 und 87°C liegt. Dadurch bleibt das medizinische Präparat auch stabil, wenn es versehentlich zu warm gelagert, transportiert oder der Sonne ausgesetzt wird. Karnaubawachs ist gesundheitlich völlig unbedenklich. Die erfindungsgemäß verwendbaren Wachse sind jedoch nicht auf die natürlichen Wachse beschränkt. Daneben können auch synthetische Wachse eingesetzt werden.
Hinzuweisen ist bei dieser Gelegenheit noch darauf, daß das als Wachs erwähnte Japanwachs zwar so bezeichnet wird, in chemischer Hinsicht aber kein Wachs ist, sondern ein wasserunlösliches Gemisch aus Pflanzenfett und freien Fettsäuren. Für die Zwecke der vorliegenden Erfindung wird es ebenfalls als Wachs bezeichnet.

Filmbildende Eigenschaften sind des weiteren z.B. von Hyprolose oder Hypromellose bekannt. Hydroxypropylmethylcellulose stellt ein Gemisch aus verschiedenen Cellulosen dar, die teilweise alkylsubstituiert sind. Ebenso eignet sich ein Polyethylenglykol mit geringer Kettenlänge, wie z.B. Magrogol 400 oder 4000, vorzugsweise als Zusatz zu Hypromellose.

Als Hilfsstoff kann beispielsweise ein Emulgator eingesetzt werden. Dies hat sich insbesondere bei der Verwendung eines Methacrylsaure-Ethylacrylat-Copolymers als polymerer Überzug als vorteilhaft erwiesen. Ein Emulgator kann Natriumdodecylsulfat sein.

Die Erfindung betrifft auch erfindungsgemäße Verwendungen des medizinischen Präparats, das vorzugsweise zur Bekämpfung und/oder Verhinderung von entzündlichen Prozessen eingesetzt werden kann. Hier ist insbesondere die Bekämpfung und/oder Verhinderung von Parodontitis zu nennen.

Die Erfindung betrifft außerdem ein erfindungsgemäßes Verfahren zur Herstellung eines bevorzugten Trägers für ein medizinisches Präparat, wie weiter oben erläutert, das zur Behandlung von entzündlichen Prozessen in der Mundhöhle geeignet ist. Bei dem Verfahren wird ein Trägernmaterial auf der Basis von Polysacchariden, vorzugsweise auf der Basis von Kohlehydraten, besonders bevorzugt in Form von Mehl, mit Wasser vermischt, bei erhöhter Temperatur reagieren gelassen und anschließend mit Feuchtigkeit versehen. Das Zuführen von Feuchtigkeit dient dazu, dem Trägermaterial die Weiterverarbeitung, z.B. zu kleinen portionsfertigen Teilen, zu erleichtern und überhaupt erst zu ermöglichen. Eine erhöhte Luftfeuchtigkeit kann dabei schon ausreichend sein, eventuell verlängert sich dadurch nur die Lagerung.

Die Erfindung betrifft des weiteren einen Träger für ein medizinisches Präparat zur Behandlung von entzündlichen Prozessen in der Mundhöhle, wie er ganz besonders nach dem genannten Verfahren erhältlich ist.

Dieser Träger kann zusammen mit einem antiphlogistischen Mittel in Form von ozonisiertem Pflanzenöl und/oder dessen Bestandteilen, und/oder mit einem antiphlogistischen Mittel in Form von Jod, insbesondere PVP-Jod, und einem Überzug das medizinische Präparat ergeben.

Im folgenden soll die Erfindung anhand von Ausführungsbeispielen näher erläutert werden.

### Beispiel 1:

### L Herstellung des flächigen Trägers

Um einen Träger herzustellen, der möglichst reizarm ist und im wesentlichen keine Nebenwirkungen oder Beeinträchtigungen mit sich bringt, wurden Weizenmehl der Type 405 und kaltes Wasser miteinander vermischt, bis ein homogener, dünnflüssiger Mehlteig entstand. Es ist genauso möglich, Mehl anderer Getreidearten, wie z.B. Dinkel zu verwenden. Auch können andere Mehltypen, d.h. andere Ausmahlungsgrade des Mehls, eingesetzt werden. Wesentlich ist, daß ein homogener, dünnflüssiger Mehlteig entsteht.
Um das medizinische Präparat mit diesem Träger auch bei an Unverträglichkeiten, wie Zöliakie, leidenden Patienten verwenden zu können, wurden erfolgreich Versuche durchgeführt, das genannte Mehl durch Maismehl zu ersetzen. Es konnte grundsätzlich bei der weiteren Herstellung genauso verfahren werden, wie nachfolgend beschrieben.
Der wie beschrieben hergestellte dünnflüssige Mehlteig wird anschließend als eine dünne Schicht in ein vorgeheiztes Backeisen gefüllt, so daß eine dünne Schicht entsteht, welche die gesamte Fläche ausfüllt. Die Teigschicht sollte frei sein von Luftblasen.
Das Backeisen wird dann geschlossen und die Teigschicht wenige Minuten backen gelassen. Nach dem Öffnen des Backeisens wird der Träger als eine größere Teigplatte erhalten. Um diese Teigplatte zur Herstellung der Träger weiterverarbeiten zu können, wird die Teigplatte nun in einen Feuchtraum gebracht oder in einen Feuchtofen. Durch die Aufnahme von Feuchtigkeit erhält sie eine Geschmeidigkeit, die es erlaubt, über eine Stanzvorrichtung mit scharfen Stanzrändern, die allgemein als Stanzeisen bezeichnet wird, jedoch nicht zwangsläufig aus diesem Material bestehen muß, Träger der gewünschten Größe zu erzeugen. Für die Zwecke dieses Ausführungsbeispiels wurden Träger in der Größe von etwa 3 x 4 mm hergestellt. Alternativ können die Träger nach dem Verlassen des Feuchtraumes oder Feuchtofens auch mit einem scharfen Messer auf eine beliebige, gewünsche Größe zugeschnitten werden.

### II. Präparierung des Trägers

Auf einem Träger, wie nach I. erhalten, wird ozonisiertes, d.h. mit Ozon angereichertes Olivenöl aufgetragen. Trägergas für das Ozon ist Luftsauerstoff.

Der so präparierte Träger wird mit einem Überzug in Form eines natürlichen Wachses versehen. Im Ausführungsbeispiel wurden hierfür Karnaubawachs und Japanwachs verwendet, um das fertige medizinische Präparat zu erhalten.
Dabei wird in bezug auf Japanwachs die hierfür übliche Bezeichnung verwendet, obwohl es sich im chemischen Sinn nicht um ein Wachs handelt. Es ist hinlänglich bekannt, daß es sich bei Japanwachs um ein wasserunlösliches Gemisch aus Pflanzenfett und freien Fettsäuren handelt.

### III. Einsetzen des medizinischen Präparats in Zahnfleischtaschen

Fünf ausgesuchten, freiwilligen Testpersonen (Probanden) mit ausgeprägter Parodontitis, bereits stark ausgeprägten Zahnfleischtaschen mit entzündlichen Herden, die unter stark empfindlichem Zahnfleisch mit teilweise erheblichem Zahnfleischbluten nach jeder Zahnreinigung litten, wurde das so hergestellte medizinische Präparat zwischen das Zahnfleisch und den Zahnhals eingesetzt
Im Gegensatz zu der üblichen Vorgehensweise wurde bei den Probanden für die Zwecke dieser Untersuchung keine zahnmedizinisch an sich erforderliche Vorbehandlung in Form einer professionellen Zahnreinigung vorgenommen, um zweifelsfrei sicherstellen zu können, daß die gefundenen Untersuchungsergebnisse auf das medizinische Präparat und nicht auf die Vorbehandlung zurückzuführen waren. Die Probanden waren sich darüber im klaren und zeigten sich mit dieser Vorgehensweise einverstanden.

Die Probanden wurden außerdem nicht darüber informiert, daß es weitere Testpersonen gab. Sie hatten untereinander keinen Kontakt, und sie kannten sich auch nicht. Die implantierten Präparate wurden täglich außer sonntags kontrolliert. Die Probanden berichteten bereits am nächsten Tag über ein subjektiv angenehmeres Mundgefühl durch ein weniger empfindliches Zahnfleisch. In Übereinstimmung damit konnte in Untersuchungen der bakteriellen Umgebung an den Implantatstellen durch tägliche vorgenommene Abstriche eine Abnahme der für die entzündlichen Prozesse verantwortlichen Matrix-Metalloproteinasen festgestellt werden.

Das Präparat hatte sich nach etwa fünf bis sieben Tagen soweit aufgelöst, daß es nicht mehr sichtbar war. Die Matrix-Metalloproteinasen wurden weiterhin überprüft. Ihre Konzentration nahm auch nach sieben Tagen noch einmal etwas ab, bis sie nach etwa zehn Tagen unterhalb der Nachweisgrenze lag. Die Probanden wurden insgesamt drei Monate lang überprüft. In dieser Zeit trat kein Zahnfleischbluten mehr auf.

### Beispiel 2:

Es wurde in allem genauso verfahren, wie in Beispiel 1, unter I. - III. angegeben, jedoch als Überzug kein natürliches Wachs bzw. Japanwachs verwendet, sondern ein Methacrylsäure-Ethylacrylat-Copolymer in einer Schichtdicke von etwa 50 µm. Dieses Copolymer wurde als Methacrylsäure-Ethylacrylat-Copolymer-(1:1) Dispersion 30% von der Merck KGaA, Darmstadt, Deutschland, bezogen.
Die Ergebnisse waren im wesentlichen dieselben, wie unter Beispiel 1 beschrieben.

### Beispiel 3a,b):

Auch in diesem Beispiel wurde in allem genauso verfahren, wie weiter oben in Beispiel 1, unter I. - III. angegeben. Auf dem Träger gleicher Größe wurde in diesem Beispiel aber nicht mit Ozon angereichertes Pflanzenöl, sondern "PVP-Jod", auch "Povidon-Jod" genannt, als Lösung aufgetragen. Unter Povidon-Jod ist ein Poly[1-vinyl-2-pyrrolidon]-Jod-Komplex zu verstehen. 100 ml der eingesetzten Lösung enthalten 10 g Povidon-Jod mit einem mittleren Molekulargewicht von 44.000 und mit einem Gehalt von 10% an verfügbarem Jod.

Es wurden zwei Testserien durchgeführt. Diese unterschieden sich dadurch, daß in der ersten als Überzug ein natürliches Wachs in Form von Karnaubawachs und zum anderen Japanwachs als Überzug verwendet wurden, und in der zweiten Testserie ein Methacrylsaüre-Ethylacrylat-Copolymer mit einer Schichtdicke von etwa 50 µm. Dieses Copolymer wurde als Methacrylsäure Ethylacrylat-Copolymer-(1:1)-Dispersion 30% von der Merck KGaA, Darmstadt, Deutschland, bezogen.
Die Ergebnisse waren im wesentlichen dieselben, wie unter Beispiel 1 beschrieben und die geringe Schwankungsbreite der Testergebnisse bei den Probanden erstaunlich.

### Beispiel 4:

Als flächiger Träger für das erfindungsgemäße medizinische Präparat wird für die Zwecke dieses Ausführungsbeispiels Cellulose in Form eines naßgelegten Vliesstoffes aus kurzen Cellulosefasern verwendet, wie sie grundsätzlich auch im Papier vorhanden sind, jedoch aufgrund der naßgelegten Herstellung nicht in der Festigkeit, wie sie das Papier aufweist. Solche Vliesstoffe sind beispielsweise von der Freudenberg & Co. KG in Weinheim Deutschland erhältlich.

Auf einem Stück dieser Cellulose in der Größe von etwa 3 x 4 mm wird ozonisiertes, d.h. mit Ozon angereichertes Olivenöl aufgetragen. Trägergas für das Ozon ist Luftsauerstoff.
Der so präparierte Träger wird mit einem Überzug in Form eines natürlichen Wachses überzogen. Im Ausführungsbeispiel wurde hierfür Karnaubawachs verwendet.

Fünf ausgesuchten, freiwilligen Testpersonen (Probanden) mit ausgeprägter Parodontitis, bereits stark ausgeprägten Zahnfleischtaschen mit entzündlichen Herden, die unter stark empfindlichem Zahnfleisch mit teilweise erheblichem Zahnfleischbluten nach jeder Zahnreinigung litten, wurde das so hergestellte medizinische Präparat zwischen das Zahnfleisch und den Zahnhals eingesetzt.
Im Gegensatz zu der üblichen Vorgehensweise wurde bei den Probanden für die Zwecke dieser Untersuchung keine zahnmedizinisch an sich erforderliche Vorbehandlung in Form einer professionellen Zahnreinigung vorgenommen, um zweifelsfrei sicherstellen zu können, daß die gefundenen Untersuchungsergebnisse auf das medizinische Präparat und nicht auf die Vorbehandlung zurückzuführen waren. Die Probanden waren sich darüber im klaren und zeigten sich mit dieser Vorgehensweise einverstanden.

Die Probanden wurden außerdem nicht darüber informiert, daß es weitere Testpersonen gab. Sie hatten untereinander keinen Kontakt, und sie kannten sich auch nicht. Die implantierten Präparate wurden täglich außer sonntags kontrolliert. Die Probanden berichteten bereits am nächsten Tag über ein subjektiv angenehmeres Mundgefühl durch ein weniger empfindliches Zahnfleisch. In Übereinstimmung damit konnte in Untersuchungen der bakteriellen Umgebung an den Implantatstellen durch tägliche vorgenommene Abstriche eine Abnahme der für die entzündlichen Prozesse verantwortlichen Matrix-Metalloproteinasen festgestellt werden.

Das Präparat hatte sich nach etwa fünf bis sieben Tagen soweit aufgelöst, daß es nicht mehr sichtbar war. Die Matrix-Metalloproteinasen wurden weiterhin überprüft. Ihre Konzentration nahm auch nach sieben Tagen noch einmal etwas ab, bis sie nach etwa zehn Tagen unterhalb der Nachweisgrenze lag. Die Probanden wurden insgesamt drei Monate lang überprüft. In dieser Zeit trat kein Zahnfleischbluteil mehr auf.

### Beispiel 5:

Als flächiger Träger für das erfindungsgemäße medizinische Präparat gemäß dieses fünften Ausführungsbeispiels wurde wieder Cellulose verwendet, wie in Beispiel 4 bereits beschrieben.
Auf einem Stück gleicher Größe wurde wieder ozonisiertes Olivenöl aufgetragen.

Der so präparierte Träger wird mit einem Überzug in Form eines Methacrylsäure-Ethylacrylat-Copolymers in einer Schichtdicke von etwa 50 µm überzogen. Dieses Copolymer wurde als Methacrylsäure-Ethylacrylat-Copolymer (1:1)-Dispersion 30% von der Merck KGaA, Darmstadt, Deutschland, bezogen.

Das so hergestellte medizinische Präparat wurde weiteren drei ausgesuchten, freiwilligen Testpersonen (Probanden) mit ausgeprägter Parodontitis, bereits stark ausgeprägten Zahnfleischtaschen mit entzündlichen Herden, die unter stark empfindlichem Zahnfleisch mit teilweise erheblichem Zahnfleischbluten nach jeder Zahnreinigung litten, zwischen das Zahnfleisch und den Zahnhals eingesetzt. Wie im ersten Ausführungsbeispiel wurde bei den Probanden keine zahnmedizinisch an sich erforderliche Vorbehandlung in Form einer professionellen Zahnreinigung vorgenommen, um eine zweifelsfreie Zuordnung der gefundenen Untersuchungsergebnisse gewährleisten Die Probanden wurden hierüber wieder ausführlich informiert und waren einverstanden.

Auch diese Probanden wußten nicht, daß es weitere Testpersonen gab. Sie hatten untereinander keinen Kontakt, und sie kannten sich auch nicht. Die implantierten Präparate wurden täglich außer sonntags kontrolliert. Die Probanden berichteten bereits am nächsten Tag über ein subjektiv angenehmeres Mundgefühl durch ein weniger empfindliches Zahnfleisch. In Übereinstimmung damit konnte in Untersuchungen der bakteriellen Umgebung an den Implantatstellen durch tägliche vorgenommene Abstriche eine Abnahme der für die entzündlichen Prozesse verantwortlichen Matrix-Metalloproteinasen festgestellt werden.

Das Präparat hatte sich nach etwa fünf bis sieben Tagen soweit aufgelöst, daß es nicht mehr sichtbar war. Die Matrix-Metalloproteinasen wurden weiterhin überprüft. Ihre Konzentration nahm auch nach sieben Tagen noch einmal etwas ab, bis sie nach etwa zehn Tagen unterhalb der Nachweisgrenze lag. Die Probanden wurden insgesamt drei Monate lang überprüft. In dieser Zeit trat kein Zahnfleischbluten mehr auf.

### Beispiel 6:

Als flächiger Träger für das erfindungsgemäße medizinische Präparat gemäß dieses sechsten Ausführungsbeispiels wurde Cellulose verwendet, wie in Beispiel 4 bereits beschrieben.

Auf einem Stück gleicher Größe wurde in diesem Beispiel "PVP-Jod", d.h. "Povidon-Jod", als Lösung aufgetragen. Wie schon in Beispiel 3a,b) erläutert, ist unter Povidon-Jod ein Poly[1-vinyl-2-pyrrolidon]-Jod-Komplex zu verstehen. 100 ml der eingesetzten Lösung enthalten 10 g Povidon-Jod mit einem mittleren Molekulargewicht von 44.000 und mit einem Gehalt von 10% an verfügbarem Jod.

Der so präparierte Träger wird mit einem Überzug in Form eines Methacrylsäure-Ethylacrylat-Copolymers in einer Schichtdicke von etwa 50 µm überzogen. Dieses Copolymer wurde als Eudragit L 30 D-55 von der Röhm GmbH, Darmstadt, Deutschland, bezogen.

Das so hergestellte medizinische Präparat wurde noch einmal weiteren drei ausgesuchten, freiwilligen Testpersonen (Probanden) mit ausgeprägter Parodontitis, bereits stark ausgeprägten Zakfleischtaschen mit entzündlichen Herden, die unter stark empfindlichem Zahnfleisch mit teilweise erheblichem Zahnfleischbluten nach jeder Zahnreinigung litten, zwischen das Zahnfleisch und den Zahnhals eingesetzt.
Wie im ersten Ausführungsbeispiel wurde bei den Probanden keine zahnmedizinisch an sich erforderliche Vorbehandlung in Form einer professionellen Zahnreinigung vorgenommen, um eine zweifelsfreie Zuordnung der gefundenen Untersuchungsergebnisse gewährleisten Die Probanden wurden hierüber wieder ausführlich informiert und waren einverstanden.

Auch diese Probanden wußten nicht, daß es weitere Testpersonen gab. Sie hatten untereinander keinen Kontakt, und sie kannten sich auch nicht. Die implantierten Präparate wurden täglich außer sonntags kontrolliert. Die Probanden berichteten bereits am nächsten Tag über ein subjektiv angenehmeres Mundgefühl durch ein weniger empfindliches Zahnfleisch. In Übereinstimmung damit konnte in Untersuchungen der bakteriellen Umgebung an den Implantatstellen durch tägliche vorgenommene Abstriche eine Abnahme der für die entzündlichen Prozesse vcrantwortlichen Matrix-Metalloproteinasen festgestellt werden.

Das Präparat hatte sich nach etwa sieben Tagen soweit aufgelöst, daß es nicht mehr sichtbar war. Die Matrix-Metalloproteinasen wurden weiterhin überprüft. Ihre Konzentration nahm auch nach sieben Tagen noch einmal etwas ab, bis sie nach etwa zehn Tagen unterhalb der Nachweisgrenze lag. Die Probanden wurden insgesamt drei Monate lang überprüft. In dieser Zeit trat kein Zahnfleischbluten mehr auf. Eine sichtbare Braunfärbung im Bereich der Implantatstelle unterblieb.

### Beispiel 7:

Beispiel 7 wurde genauso durchgeführt, wie Beispiel 6, es wurde jedoch als Überzug wieder ein natürliches Wachs in Form von Karnaubawachs verwendet. Nennenswerte Unterschiede der Testergebnisse bei den nun untersuchten weiteren zwei Testpersonen ergaben sich nicht.

### Beispiele 8 - 10:

Die Beispiele 1 bis 3a,b) wurden wiederholt, jedoch nur jeweils zwei Testpersonen unter den gleichen Bedingungen, wie weiter oben geschildert, überprüft. Es wurde jeweils ein Stück des Trägers verwendet, der hergestellt war, wie in Beispiel 1, unter I., näher beschrieben. Dieser wurde nun auf eine Größe von etwa 4 x 5 mm geschnitten oder gestanzt. Signifikante Veränderungen konnten im Hinblick auf die beobachteten Versuchsergebnisse nicht festgestellt werden.

### Beispiele 11 - 14:

Die Beispiele 4 bis 7 wurden wiederholt, jedoch nur jeweils zwei Testpersonen unter den gleichen Bedingungen, wie weiter oben geschildert, überprüft. Es wurde jeweils ein Stück der schon beschriebenen Cellulose in der Größe von etwa 4 x 5 mm verwendet. Signifikante Veränderungen konnten im Hinblick auf die beobachteten Versuchsergebnisse nicht festgestellt werden.

## Patentansprüche

1. Medizinisches Präparat, bei dem ein antiphlogistisches Mittel in Form von ozonisiertem Pflanzenöl und/oder dessen Bestandteilen auf einem Trägermaterial auf pflanzlicher und/oder Kunststoffbasis aufgebracht ist, und das mit einem polymeren Überzug oder einem Überzug aus einem natürlichen oder synthetischen Wachs versehen ist, wobei der Träger und der Überzug im wesentlichen biologisch abbaubar sind.

2. Medizinisches Präparat nach Anspruch 1, **dadurch gekennzeichnet, daß** das Trägermaterial auf der Basis von Polysacchariden aufgebaut ist.

3. Medizinisches Präparat nach Anspruch 1 oder 2, **dadurch gekennzeichnet, daß** das Trägermaterial auf der Basis von Stoffen aufgebaut ist, die ausgewählt sind aus Stärke, Cellulose, Cellulosederivaten, Polyglycoliden und deren Copolymeren, Polyvinylpyrrolidon und Mischungen davon.

4. Medizinisches Präparat nach einem der Ansprüche 1 bis 3, **dadurch gekennzeichnet, daß** das ozonisierte Pflanzenöl ausgewählt ist aus Rapsöl, Sonnenblumenkernöl, Sojaöl, Rizinusöl, Olivenöl, Leinöl, Kokosöl, Palmöl, zumindest einem Bestandteil, Derivat in Form eines Esters, vorzugsweise ein Glycerinester einer mehrfach ungesättigien Fettsäure, und/oder Mischungen davon.

5. Medizinisches Präparat nach einem der Ansprüche 1 bis 4, **dadurch gekennzeichnet, daß** der Träger und der Überzug eine unterschiedliche Abbaugeschwindigkeit aufweisen.

6. Medizinisches Präparat nach einem der Ansprüche 1 bis 5, **dadurch gekennzeichnet, daß** der polymere Überzug ausgewählt ist aus einem natürlichen Wachs, Cellulosederivaten, Polyethylenglykol, (Meth-)acrylsäurecopoly-meren und/oder Mischungen davon sowie gegebenenfalls weiteren Hilfsstoffcn.

7. Medizinisches Präparat nach einem der Ansprüche 1 bis 6, **dadurch gekennzeichnet, daß** das in dem Pflanzenöl enthaltene Ozon seinerseits Sauerstoff als Trägergas aufweist.

8. Verwendung von ozonisiertem Pflanzenöl und/oder dessen Bestandteilen zur Herstellung des medizinischen Präparats nach einem der Ansprüche 1 bis 7 zur Behandlung von entzündlichen Prozessen.

9. Verwendung nach Anspruch 8 zur Behandlung von entzündlichen Prozessen in der Mundhöhle, insbesondere zur Bekämpfung und/oder Verhinderung von Parodontitis.

10. Verfahren zur Herstellung eines medizinischen Präparats zur Behandlung von entzündlichen Prozessen in der Mundhöhle, bei dem zunächst ein Träger hergestellt wird, indern ein Trägermaterial auf der Basis von Polysacchariden, vorzugsweise auf der Basis von Kohlehydraten, besonders bevorzugt in Form von Mehl, mit Wasser vermischt, bei erhöhter Temperatur reagieren gelassen und anschließend mit Feuchtigkeit versehen wird, und anschließend der so erhaltene Träger mit einem antiphlogistischen Mittel in Form von ozonisiertem Pflanzenöl und/oder dessen Bestandteilen, und/oder mit einem antiphlogistischen Mittel in Form von Jod, insbesondere PVP-Jod, und einem Überzug versehen wird.

## Claims

1. Medical preparation in which an anti-inflammatory agent in the form of ozonized plant oil and/or its components is applied to a substrate made of a plant and/or synthetic base, and that features a polymeric coating or a coating made of a natural or synthetic wax, where the carrier and the coating are basically biodegradable.

2. Medical preparation according to Claim 1, **characterised in that** the substrate is built up on the basis of polysaccharides.

3. Medical preparation according to Claim 1 or 2, **characterised in that** the substrate is built up on the basis of substances that are selected from starch, cellulose, cellulose derivatives, polyglycolides and their co-polymers, polyvinylpyrrolidone, or mixtures thereon.

4. Medical preparation according to one of the Claims 1 to 3, **characterised in that** the ozonized plant oil is selected from rapeseed oil, sunflower seed oil, soybean oil, castor oil, olive oil, linseed oil, coconut oil, palm oil, at least one component, a derivative in the form of au ester, preferably a glycerol ester of a polyunsaturated fatty acid, and/or mixtures thereof.

5. Medical preparation according to one of the Claims I to 4, **characterised in that** the carrier and the coating each have a different rate of decomposition.

6. Medical preparation according to one of the Claims 1 to 5, **characterised in that** the polymeric coating is selected from a natural wax, cellulose derivatives, polyethylene glycol, (meth-)acrylic acid copolymers and/or mixtures thereof, as well as possibly other auxiliary substances.

7. Medical preparation according to one of the Claims 1 to 6, **characterised in that** the ozone contained in the plant oil bears oxygen as carrier gas.

8. Use of ozonized plant oil and/or its components to produce the medical preparation according to one of the Claims 1 to 7 for the treatment of inflammatory processes.

9. Use according to Claim 8 for the treatment of inflammatory processes in the oral cavity, in particular for combating and/or preventing periodontitis.

10. Method for manufacturing a medical preparation for the treatment of inflammatory processes in the oral cavity, in which firstly a carrier is manufactured by taking a substrate on the basis of polysaccharides, preferably on the basis of carbohydrates, and what is specially preferred in the form of flour, mixed with water, and allowing it to react at an elevated temperature, then adding moisture to it, and then to the carrier obtained in this way there is added an anti-inflammatory agent in the form of an ozonized plant oil and/or its components, and/or an anti-inflammatory agent in the form of iodine, in particular PVP-I, as well as a coating.

## Revendications

1. Préparation médicale dans laquelle un moyen anti-infiammatoire sous la forme d'une huile végétale traitée l'ozone et/ou de ses composants est appliqué sur un matériau support à base de plantes ou de matière plastique, et qui est muni d'un revêtement polymère ou un revêtement en cire naturelle ou synthétique, le support et le revêtement étant pour l'essentiel dégradables.

2. Préparation médicale selon la revendication 1, **caractérisée en ce que** le matériau support est constitué sur la base de polysaccharides.

3. Préparation médicale selon la revendication 1 ou 2, **caractérisée en ce que** le matériau support est constitué sur la base de matériaux qui sont choisis parmi l'amidon, la cellulose, des dérivés de cellulose, de polyglycolides et leurs copolymères, la polyvinylpyrrolidone et des mélanges de ceux-ci.

4. Préparation médicale selon l'une des revendications 1 à 3, **caractérisée en ce que** l'huile végétale traitée à l'ozone est choisie parmi l'huile de colza, l'huile de graines de tournesol, l'huile de soja, l'huile de ricin, l'huile d'olive, l'huile de lin, l'huile de coco, l'huile de palme, au moins un composant, dérivé sous la forme d'un ester, de préférence un ester de glycérine d'un acide gras polyinsaturé, et/ou des mélanges de ceux-ci.

5. Préparation médicale selon l'une des revendications 1 à 4, **caractérisée en ce que** le support et le revêtement présentent des vitesses de dégradation différentes.

6. Préparation médicale selon l'une des revendications 1 à 5, **caractérisée en ce que** le revêtement polymère est choisi parmi une cire naturelle, des dérivés de cellulose, le polyéthylène glycol, des copolymères d'acide méthacrylique et/ou des mélanges de ceux-ci ainsi que le cas échéant d'autres excipients.

7. Préparation médicale selon l'une des revendications 1 à 6, **caractérisée en ce que** l'ozone contenu dans l'huile végétale présente à son tour de l'oxygène comme gaz porteur.

8. Utilisation d'huile végétale traitée à l'ozone et/ou de ses constituants pour la fabrication de la préparation médicale selon l'une des revendications 1 à 7 pour le traitement de processus inflammatoires.

9. Utilisation selon la revendication 8 pour le traitement de processus inflammatoires dans la cavité buccale, notamment pour la lutte contre et/ou la prévention de la parodontose.

10. Procédé pour la fabrication d'un support pour une préparation médicale pour le traitement de processus inflammatoires dans la cavité buccale, dans lequel un matériau support à base de polysaccharides, de préférence à base d'hydrate de carbone, et plus particulièrement sous forme de farine, est mélangé à de l'eau, est mis à réagir à température élevée puis est soumis à l'humidité, et le support ainsi obtenu est pourvu d'un moyen anti-inflammatoire sous la forme d'une huile végétale traitée à l'ozone et/ou de ses composants, et/ou d'un moyen anti-inflammatoire sous la forme d'iode, notamment de la PVP iodée, et d'un revêtement.
